# EUROPEAN PATENT APPLICATION

(11) **EP 4 039 246 A1**
(43) Date of publication of application: **10.08.2022**
(21) Application number: 20872325.4
(22) Date of filing: 28.09.2020
(51) Int. Cl.: A61K 8/73, A61K 8/04, A61K 8/92, A61Q 1/00, A61Q 5/00, B01F 17/00, B01J 13/00

(54) **EMULSION COMPOSITION, COSMETIC COMPOSITION, AND METHOD FOR PRODUCING EMULSION COMPOSITION**

(30) Priority: 03.10.2019 JP 2019183252
(71) Applicant: Daio Paper Corporation, Ehime 799-0492 (JP)
(72) Inventor: SASAKI, Hiroto, Shikokuchuo-shi, Ehime 799-0492 (JP)
(74) Representative: Held, Stephan
(86) International application number: PCT/JP2020/036600
(87) International publication number: WO 2021/065788

(57) **Abstract**

Provided are an emulsion composition, a cosmetic composition, and a method for preparing an emulsion composition, in which the oil components are not limited to vegetable oils, and which do not require xanthan gum or welan gum as an essential component. The emulsion composition contains an oil component, an aqueous dispersion medium, an emulsifier, and CNF. The CNF is an emulsion stabilizer, and obtained by unmodified cellulose defibration, and the emulsifier amount is less than 20 mass% of the total composition. The cosmetic composition contains the emulsion composition. The method includes preparing an oil phase, preparing an aqueous phase, and emulsifying by mixing the oil and aqueous phases. CNF obtained by defibrating unmodified cellulose is added in at least preparing an aqueous phase or emulsifying, and an emulsifier is added in at least preparing an oil phase, preparing an aqueous phase, or emulsifying.

## Description

### Technical Field

The present invention relates to an emulsion composition, a cosmetic composition, and a method for preparing an emulsion composition.

### Background Art

In cosmetics, such as cream, emulsion, liquid foundation, and hair conditioner, coating materials, such as paint, foods, such as mayonnaise and dressing, or the like products, when oil components and an aqueous medium are mixed, emulsification (emulsion) technique is employed for avoiding separation of the oil phase and the aqueous phase. Recently, as an effective use of biomass-derived cellulose, cellulose nanofibers (CNF) in which nano-level-sized fibers are dispersed has been receiving attention. Development of the cellulose nanofibers are expected in the fields of the above-mentioned cosmetics, coating materials, foods, and the like, for their high static viscosity and thixotropic properties. However, not many reports or proposals have been made on emulsions containing cellulose nanofibers.

For example, Patent Literature 1 proposes emulsions using cellulose nanofibers (dispersion liquid) and an emulsification method. This proposal is to add cellulose nanofibers (dispersion liquid) as an emulsifier to emulsify under mechanical shear force. However, oil components which can be used in this proposal are limited to natural vegetable oils, such as rapeseed oil or olive oil. It is assumed that vegetable oils have carboxylic acid and affinity to cellulose, so that cellulose nanofibers act as an emulsifier. However, use of oil components without a hydrophilic group is also demanded.

Patent Literature 2 proposes an oil-in-water external emulsion composition. This proposal is to use fermented cellulose and/or cellulose nanofibers in combination with xanthan gum and/or welan gum in a particular amount with respect to the cellulose. However, such combined use of a particular amount of xanthan gum and/or welan gum extremely limits the applications, and proposal of other solutions is desired.

### Prior Art Publication

### Patent Literature

Patent Literature 1: JP 2015-157796 A
Patent Literature 2: JP 2017-222594 A
Patent Literature 2: JP 2019-156824 A

### Summary of the Invention

### Problems to be Solved by the Invention

It is a primary object of the present invention to provide an emulsion composition, a cosmetic composition, and a method for preparing an emulsion composition, in which oil components are not limited to vegetable oils, and which do not require a polysaccharide thickener, such as xanthan gum or welan gum, as an essential component.

### Means for Solving the Problem

The above-mentioned problems are solved by an emulsion composition containing an oil component, an aqueous dispersion medium, an emulsifier, and cellulose nanofibers, wherein the cellulose nanofibers are an emulsion stabilizer, and formed of defibrated unmodified cellulose, and an amount of the emulsifier is less than 20 mass% of a total amount of the emulsion composition.

In this regard, as a proposal regarding an emulsion composition, for example, there is proposed that cellulose nanofibers are "obtained by chemically processing cellulose raw material to obtain chemically-modified (carboxymethylated, carboxylated, phosphoesterified, cationized, or the like) cellulose (modified cellulose), followed by defibration" (JP 2019-156824 A, Patent Literature 3). This proposal is "to provide an emulsifier composition having excellent emulsion stability, in particular, prolonged emulsion stability". In sum, the proposal conclude that use of modified cellulose is preferred for emulsion stability. However, the present inventor has aimed to eliminate limitation (restriction) on oil components, and has found out that, from this perspective, cellulose is more preferably unmodified. Yet, the present inventor has also found out that mere unmodification of cellulose is not always preferred. Through various researches, the present inventor has revealed the difference in role between an emulsifier and cellulose nanofibers, to thereby reach the above-mentioned solution.

### Effect of the Invention

According to the present invention, there is provided an emulsion composition, a cosmetic composition, and a method for preparing an emulsion composition, in which the oil components are not limited to vegetable oils, and which do not require a polysaccharide thickener, such as xanthan gum or welan gum, as an essential component.

### Brief Description of the Drawings

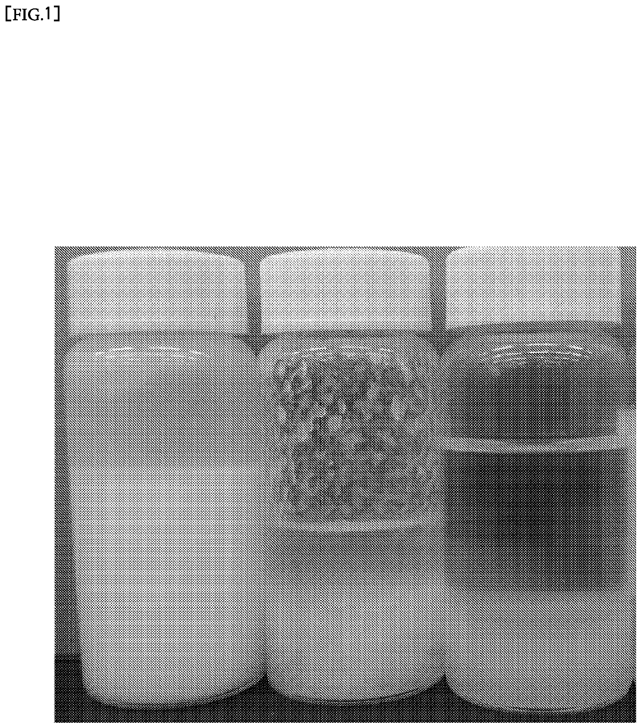

Fig. 1 is a photograph showing the emulsion states.

### EMBODIMENTS OF THE INVENTION

Aspects for carrying out the present invention will now be explained. Note that the embodiments are mere examples of the present invention, and the scope of the present invention is not limited to the scope of the present embodiments.

The emulsion composition according to the present embodiment contains an oil component, an aqueous dispersion medium, an emulsifier, and unmodified cellulose nanofibers. In the present embodiment, the emulsifier acts to improve compatibility between the oil component and the aqueous medium for emulsification. On the other hand, the unmodified cellulose nanofibers act as a stabilizer after the emulsification (emulsion stabilizer). These facts enable emulsification of an aqueous medium and an oil component other than vegetable oils, which has hitherto not been intended. In addition, phase separation (separation of an aqueous phase and an oil phase) may be prevented over time, and emulsion stabilization may be maintained.
Details are discussed below.

### (Applications)

The emulsion composition according to the present embodiment may be used as a component of, for example, cosmetic compositions, intermediate materials of medicine, food materials, paints, resins, or the like, and is particularly suitable as a component of cosmetic compositions.

Here, the cosmetic compositions may be used, for example, as skin cosmetics, hair cosmetics, or the like. The skin cosmetics may include, for example, toner, emulsion, cold cream, vanishing cream, massage cream, emollient cream, cleansing cream, beauty serum, facial mask, foundation, sunscreen cosmetics, suntan cosmetics, moisturizing cream, hand cream, whitening emulsion, and various lotions.

The hair cosmetics may include, for example, shampoo, rinse, hair conditioners, two-in-one shampoo, hair styling products (hair foam, hairdressing gel, or the like), hair treatment (hair cream, treatment lotion, or the like), hair color, and hair tonic or hair grower lotions.

Further, the cosmetic compositions may include, for example, pre-shave lotion, after-shave lotion, dentifrice, ointment, patches, cleaners like hand cleaner, and fragrance.

Note that the emulsion composition of the present embodiment is excellent in emulsion stability, and its application is not limited to cosmetic compositions.

### (Oil Component)

The oil component which may be contained in the emulsion composition of the present embodiment is not limited to vegetable oils. The oil component may be, for example, oils and fats, higher alcohols, higher fatty acids, esters, and hydrocarbons. In the Examples to be discussed later, examples of use of caster oil, dimethyl silicone oil, ethylhexyl palmitate, and heptane will be discussed.

When the emulsion composition of the present embodiment is to be used as a component of a cosmetic composition, the oil component preferably contains one or more hydrocarbons selected from the group consisting of squalene, paraffin, polyethylene wax, microcrystalline wax, liquid paraffin, and mineral oils.

Further, the oil component may be one or more members selected from the group consisting of, for example, natural animal and vegetable oils and fats, such as jojoba oil, macadamia nut oil, avocado oil, evening primrose oil, mink oil, rapeseed oil, caster oil, sunflower seed oil, corn oil, cacao oil, palm oil, rice bran oil, olive oil, almond oil, sesame oil, safflower oil, soybean oil, camelia oil, Prunus armeniaca kernel oil, caster oil, mink oil, cottonseed oil, Japan wax, palm oil, palm kernel oil, egg-yolk oil, lanolin, and squalene; hydrocarbons, such as synthesized triglyceride, squalane, liquid paraffin, petrolatum, ceresin, microcrystalline wax, and isoparaffin; waxes, such as carnauba wax, paraffin wax, spermaceti, beeswax, candelilla wax, and lanolin; higher alcohols, such as cetanol, stearyl alcohol, lauryl alcohol, cetostearyl alcohol, oleyl alcohol, behenyl alcohol, lanolin alcohol, hydrogenated lanolin alcohol, hexyldecanol, and octyldodecanol; higher fatty acids, such as lauric acid, myristic acid, palmitic acid, stearic acid, behenic acid, isostearic acid, oleic acid, linoleic acid, linolic acid, oxystearic acid, undecylenic acid, lanolin fatty acid, hard lanolin fatty acid, and soft lanolin fatty acid; cholesterol and derivatives thereof, such as cholesteryl-, octyldodecyl-, and behenyl-cholesterols; esters, such as isopropyl myristate, isopropyl palmitate, isopropyl stearate, glycerol 2-ethylhexanoate, and butyl stearate; polar oils, such as diethylene glycol monopropyl ether, polyoxyethylene polyoxypropylene pentaerythritol ether, polyoxypropylene butyl ether, and ethyl linoleate; and silicones including various derivatives thereof, such as amino-modified silicone, epoxy-modified silicone, carboxymodified silicone, carbinol-modified silicone, methacrylmodified silicone, mercapto-modified silicone, phenolmodified silicone, single-end reactive silicone, heterofunctional group-modified silicone, polyethermodified silicone, methylstyryl-modified silicone, alkylmodified silicone, higher fatty acid ester-modified silicone, special modified hydrophilic silicone, higher alkoxy-modified silicone, higher fatty acid-containing silicone, and fluorine-modified silicone, more specifically, silicone resin, methylphenylpolysiloxane, methylpolysiloxane, octamethylcyclotetrasiloxane, decamethylcyclopentasiloxane, dodecamethylcyclohexanesiloxane, methylcyclopolysiloxane, octamethyltrisiloxane, decamethyltetrasiloxane, polyoxyethylene-methylpolysiloxane copolymers, polyoxypropylene-methylpolysiloxane copolymers, poly(oxyethylene-oxypropylene) methylpolysiloxane copolymers, methyl hydrogen polysiloxane, tetrahydro tetramethylcyclotetrasiloxane, stearoxymethylpolysiloxane, cetoxymethylpolysiloxane, methylpolysiloxane emulsion, highly-polymerized methylpolysiloxane, trimethylsiloxy silicate, cross-linked methylpolysiloxane, cross-linked methylphenylpolysiloxane, and cross-linked methylphenylpolysiloxane.

Recently, cosmetic liquid (emulsified liquid) with a higher water (aqueous phase) content, so called water-rich formula, has been developed. In such cosmetic liquid, content of the oil components is as low as about 10 to 20 mass%. As cosmetic liquid having such a low content of oil components is easily emulsified, problems in emulsification may not be significant. Thus, the effect of the emulsion composition according to the present embodiment may be more pronounced when the content of the oil components is higher, for example, 20 mass% or more, particularly 30 mass% or more.

### (Aqueous Medium)

The aqueous medium in the emulsion composition according to the present embodiment may be water alone, or a mixed liquid of water and a prescribed component.

The prescribed component mixed with water may be, for example, water-soluble alcohols, such as ethanol or isopropanol; or hydrophilic polyhydric alcohols, such as glycerin, ethylene glycol, or butanediol.

### (Emulsifier)

The emulsion composition according to the present embodiment contains an emulsifier separate from the cellulose nanofibers or a dispersion of cellulose nanofibers.

The emulsifier may be, for example, nonionic surfactants, anionic surfactants, cationic surfactants, amphoteric surfactants, or phospholipid, and ester-type or ester-ether-type nonionic surfactants are preferred. As an ester-type or ester-ether-type nonionic surfactant, the emulsifier may be selected from wide varieties, so that designing for improved initial dispersibility of the oil phase may be facilitated, and designing of the emulsion composition, for example, for controlling texture or fluidity of the emulsion composition, may be facilitated.

The nonionic surfactants may be, for example, glycerin fatty acid esters, polyglycerin fatty acid esters, propylene glycol fatty acid esters, sorbitan fatty acid esters, or fatty acid esters of sorbitol, or alkylene glycol addition products thereof, polyalkylene glycol fatty acid esters, sucrose fatty acid esters, polysorbate 20, polysorbate 60, polysorbate 80, polyoxyalkylene alkyl ethers, or polyoxyethylene alkylphenyl ethers.

The nonionic surfactants may also preferably be, for example, polyoxyethylene glycerin fatty acid esters, polyglycerin fatty acid esters, sorbitan fatty acid esters, polyoxyethylene sorbitan fatty acid esters, polyoxyethylene sorbit fatty acid esters, polyethylene glycol fatty acid esters, polyoxyethylene caster oil, polyoxylethylene hydrogenated caster oil, polyoxyethylene phytosterol, polyoxyethylene polyoxypropylene alkyl ethers, polyoxyethylene lanolin, polyoxyethylene lanolin alcohol, polyoxyethylene beeswax derivatives, polyoxyethylene alkylamines, polyoxyethylene fatty acid amides, polyoxyethylene alkylphenyl formaldehyde condensates, or polyoxyethylene alkyl ether phosphate (salt).

Further, as far as the effects of the present invention is not impaired, mild surfactants may also be used, including anionic surfactants, such as alkyl sulfates, polyoxyethylene alkyl sulfates, alkylbenzene sulfonates, or α-olefin sulfonates; cationic surfactants, such as alkyltrimethylammonium chloride, dialkyldimethylammonium chloride, or benzalkonium chloride; amphoteric surfactants, such as alkyldimethylaminoacetic acid betaine or alkylamidodimethylaminoacetic acid betaine; surface active natural products, such as lecithin, lanolin, cholesterol, or saponin; sulfosuccinates; or ethylene oxide-propylene oxide block copolymers.

The content of the emulsifier is preferably less than 20 mass%, more preferably 15 mass% or less, particularly preferably 10 mass% or less, of the total amount of the emulsion composition. With 20 mass% or more emulsifier, emulsion stability tends to be poor. This is assumed to be because an excess amount of emulsifier tends to lower the fluidity or cause gelation of the aqueous phase containing the cellulose nanofibers, resulting in reduced homogenous dispersibility of the aqueous phase and the oil phase.

The content of the cellulose nanofibers (in terms of absolute dry solid) may be adjusted to preferably more than 2.0 mass%, more preferably 2.1 mass% or more, particularly preferably 2.2 mass% or more, of the content of the emulsifier by controlling the contents of the emulsifier and/or cellulose nanofibers. The content of the cellulose nanofibers (2.0 mass% dispersion) may be adjusted to preferably more than (over 100 mass%), more preferably 105 mass% or more of, particularly preferably 110 mass% or more of, the content of the emulsifier by controlling the contents of the emulsifier and/or the cellulose nanofibers. With too small a content of the cellulose nanofibers with respect to the content of the emulsifier, dispersibility of the aqueous phase containing the cellulose nanofibers may be lowered, and particularly, the cellulose may be prone to aggregation or gelation.

Note that, for example, glycerin, propylene glycol, and behenyl alcohol may be used in some cases as an aid of moisturizers, viscosity modifiers, or the like, but also acts as an emulsifier. In view of this, according to the present embodiment, the above-mentioned aids are included in the emulsifier in calculating the content of the emulsifier.

### (Cellulose Nanofibers)

The emulsion composition according to the present embodiment contains unmodified cellulose nanofibers as an emulsion stabilizer (emulsification aid) apart from the emulsifier, in the form of a dispersion, as needed. The cellulose nanofibers will be discussed in detail below.

As cellulose raw material (also referred to as "raw material pulp" hereinbelow), for example, one or more members may be selected from the group consisting of wood pulp made from hardwood, softwood, or the like; non-wood pulp made from straw, bagasse, cotton, hemp, bast fibers, or the like; and de-inked pulp (DIP) made from recovered used paper, waste paper, or the like. These various raw materials may be in the form of, for example, a ground product (powdered product), such as so called cellulosebased powder.

In this regard, however, it is preferred to use wood pulp rather than non-wood pulp or de-inked pulp, as contamination of impurities may be avoided as much as possible and a higher content of α-cellulose may be obtained, which is, among cellulose components, insoluble in alkali. The wood pulp may be, for example, one or more members selected from the group consisting of chemical pulp, such as hardwood kraft pulp (LKP) and softwood kraft pulp (NKP), and mechanical pulp (TMP).

The hardwood kraft pulp may be hardwood bleached kraft pulp, hardwood unbleached kraft pulp, or hardwood semi-bleached kraft pulp. Similarly, the softwood kraft pulp may be softwood bleached kraft pulp, softwood unbleached kraft pulp, or softwood semi-bleached kraft pulp.

The mechanical pulp may be, for example, one or more member selected from the group consisting of stone ground pulp (SGP), pressure stone ground pulp (PGW), refiner ground pulp (RGP), chemi-ground pulp (CGP), thermo-ground pulp (TGP), ground pulp (GP), thermomechanical pulp (TMP), chemithermomechanical pulp (CTMP), refiner mechanical pulp (RMP), and bleached thermomechanical pulp (BTMP).

According to the present embodiment, it is preferred that the cellulose raw material is unmodified, i.e., has not been subjected to chemical modification, such as TEMPO-oxidation, modification with oxoacid of phosphorus, such as phosphoric acid or phosphorus acid, or carbamate modification. In this regard, if the cellulose raw material is chemically modified, in general, the cellulose nanofibers resulting from the subsequent defibration is highly homogeneous. In particular, according to the present embodiment, improved homogeneity is an important factor of emulsion stability in view of the low content of the cellulose nanofibers with respect to the total amount of the emulsion composition. However, with the chemically -modified cellulose, emulsion stability of the emulsion (mixed liquid) is poor under the influence of electrostatic repulsion. Thus, according to the present embodiment wherein cellulose nanofibers are used as an emulsion stabilizer, unmodified cellulose is preferred. With the unmodified cellulose nanofibers, gelatinization of the emulsion composition may be prevented, and in the application to a cosmetic composition, sensation in use may be improved, e.g., gooey texture is restricted. Note that, as used herein, being unmodified is defined as absence of modification of the hydroxyl groups on cellulose surface prior to defibration.

The cellulose raw material is defibrated into cellulose nanofibers, which are usually obtained in the form of a dispersion.

Prior to the defibration into the cellulose nanofibers, pretreatment may be performed by a chemical method. Such pretreatment by a chemical method may be, for example, hydrolysis of polysaccharides with acid, such as sulfuric acid (acid treatment), or hydrolysis of polysaccharides with enzyme (enzyme treatment).

The pretreatment may preferably be performed at least either of the acid treatment or the enzyme treatment. These treatments may result in lower water retention, high degree of crystallinity, and also high homogeneity of the cellulose nanofibers. In this regard, cellulose nanofibers at a low water retention are easily dewatered, so that dewaterability of the dispersion (slurry) of the cellulose nanofibers may be improved. Further, the amorphous region of hemicellulose and cellulose in the pulp (cellulose raw material) may be decomposed, which leads to reduction of energy required for the treatment to make the raw material finer and improvement in uniformity and dispersibility of the cellulose fibers. The dispersibility of the cellulose fibers serves, for example, improvement in homogeneity of the cellulose nanofibers. However, the pretreatment lowers the embodiment ratio of cellulose nanofibers, and it is thus preferred to avoid excessive pretreatment.

As an enzyme used in the enzyme treatment, preferably at least one of, more preferably both of cellulase enzymes and hemicellulase enzymes are used. Such enzymes facilitate the defibration. It is noted that cellulase enzymes cause decomposition of cellulose in the presence of water, whereas hemicellulase enzymes cause decomposition of hemicellulose in the presence of water.

The cellulase enzymes may be enzymes produced by, for example, the genus Trichoderma (filamentous fungus), the genus Acremonium (filamentous fungus), the genus Aspergillus (filamentous fungus), the genus Phanerochaete (basidiomycete), the genus Trametes (basidiomycete), the genus Humicola (filamentous fungus), the genus Bacillus (bacteria), the genus Schizophyllum (basidiomycete), the genus Streptomyces (bacteria), and the genus Pseudomonas (bacteria). These cellulase enzymes are available as reagents or commercial products. Examples of the commercial products may include, for example, Cellulosin T2 (manufactured by HBI ENZYMES INC.), Meicelase (manufactured by MEIJI SEIKA PHARMA CO., LTD.), Novozyme 188 (manufactured by NOVOZYMES), Multifect CX10L (manufactured by GENENCOR), and cellulase enzyme GC220 (manufactured by GENENCOR).

The cellulase enzymes may also be either EG (endoglucanase) or CBH (cellobiohydrolase). EG and CBH may be used alone or in mixture, or further in mixture with hemicellulase enzymes.

The hemicellulase enzymes may be, for example, xylanase, which decomposes xylan; mannase, which decomposes mannan; and arabanase, which decomposes araban. Pectinase, which decomposes pectin, may also be used.

Hemicellulose is a polysaccharide other than pectin, which is present between cellulose microfibrils of plant cell walls. Hemicellulose has wide varieties and varies depending on the kinds of wood and among cell wall layers. Glucomannan is a major component in the secondary walls of softwood, whereas 4-O-methylglucuronoxylan is a major component in the secondary walls of hardwood. Thus, use of mannase is preferred for obtaining fine fibers from softwood bleached kraft pulp (NBKP), whereas use of xylanase is preferred for obtaining fine fibers from hardwood bleached kraft pulp (LBKP).

The amount of the enzyme to be added with respect to the amount of the cellulose raw material may depend on, for example, the kind of enzyme, the kind of wood (either softwood or hardwood) used as a raw material, or the kind of mechanical pulp. The amount of the enzyme to be added may preferably be 0.1 to 10 mass%, more preferably 0.2 to 5 mass%, particularly preferably 0.3 to 3 mass%, of the amount of the cellulose raw material. With the amount of the enzyme below 0.1 mass%, sufficient effect due to the addition of the enzyme may not be obtained. With the amount of the enzyme over 10 mass%, the cellulose may be saccharified to lower the yield of the fine fibers. A problem also resides in that improvement in effect worth the increased amount added may not be observed.

When a cellulase enzyme is used as the enzyme, the enzyme treatment is preferably carried out at a pH in a weakly acidic region (pH = 3.0 to 6.9) in view of the enzymatic reactivity. On the other hand, when a hemicellulase enzyme is used as the enzyme, the enzyme treatment is preferably carried out at a pH in a weakly alkaline region (pH = 7.1 to 10.0).

Whether a cellulase enzyme or a hemicellulase enzyme is used, the enzyme treatment is carried out at a temperature of preferably 30 to 70 °C, more preferably 35 to 65 °C, particularly preferably 40 to 60 °C. At a temperature of 30 °C or higher, the enzymatic activity is hard to be lowered, and prolongation of the treatment time may be avoided. At a temperature of 70 °C or lower, enzyme inactivation may be avoided.

The duration of the enzyme treatment may depend on, for example, the type of the enzyme, the temperature in the enzyme treatment, and the pH in the enzyme treatment. Generally, the duration of the enzyme treatment is 0.5 to 24 hours.

The enzyme treatment is preferably followed by inactivation of the enzymes. Inactivation of enzymes may be effected by, for example, addition of an alkaline aqueous solution (preferably at pH 10 or higher, more preferably at pH 11 or higher) or addition of 80 to 100 °C hot water.

Incidentally, an alkali treatment prior to the defibration causes partial dissociation of hydroxyl groups in hemicellulose or cellulose in pulp, resulting in anionization of the molecules, which weakens intra- and intermolecular hydrogen bonds to promote dispersion of cellulose fibers during the defibration. However, modification of cellulose is not preferred according to present embodiment, as discussed above.

The defibration of the raw material pulp may be performed by beating the raw material pulp in, for example, beaters, homogenizers, such as high-pressure homogenizers and high-pressure homogenizing apparatus, millstone friction machines, such as grinders and mills, single-screw kneaders, multi-screw kneaders, kneaders, refiners, and jet mills. It is preferred to use refiners or jet mills.

The defibration of the raw material pulp is preferably effected so that the average fiber diameter, average fiber length, water retention, degree of crystallinity, peak value of a pseudo particle size distribution curve of the resulting cellulose nanofibers, the pulp viscosity, the degree of polymerization, and the B-type viscosity of the dispersion fall under the desired values or evaluations to be discussed below.

The average fiber diameter (average fiber width, or average of diameters of single fibers) of the cellulose nanofibers is preferably 10 to 1000 nm, more preferably 10 to 100 nm, particularly preferably 10 to 80 nm. With an average fiber diameter of the cellulose nanofibers below 10 nm, the viscosity of the cellulose nanofiber dispersion is excessively high, which proportionally makes the viscosity of the emulsion composition high, so that a desired amount of cellulose nanofibers may not be contained.

On the other hand, with an average fiber diameter of the cellulose nanofibers above 1000 nm, fluidity of the emulsion composition may be impaired and unpleasant texture may be imparted.

The average fiber diameter of the cellulose nanofibers may be adjusted by, for example, selection, pretreatment, or defibration of the raw material pulp.

The average fiber diameter of cellulose nanofibers may be determined by the following process.

First, 100 ml of an aqueous dispersion (slurry) of cellulose nanofibers having a solid concentration of 0.01 to 0.1 mass% is filtered through a TEFLON (registered trademark) membrane filter, and subjected to solvent substitution once with 100 ml of ethanol and three times with 20 ml of t-butanol. Then the resulting mass is lyophilized and coated with osmium to obtain a sample. An electron microscopic SEM image of this sample is observed at a magnification of 3000 to 30000 folds, depending on the width of the constituent fibers. Specifically, two diagonal lines are drawn on the observation image, and three arbitrary straight lines passing the intersection of the diagonals are drawn. Then, the widths of a total of 100 fibers crossing these three straight lines are visually measured. The median diameter of the measured values is taken as the average fiber diameter.

The average fiber length (average of lengths of single fibers) of the cellulose nanofibers is preferably 0.3 to 200 µm, more preferably 0.4 to 200 µm, particularly preferably 0.5 to 200 µm. With an average fiber length of the cellulose nanofibers above 200 µm, the fibers may easily aggregate to cause deterioration of fluidity, i.e., deterioration of texture, of the emulsion composition.

The average fiber length of the cellulose nanofibers may be adjusted by, for example, selection, pretreatment, or defibration of the raw material pulp.

The average fiber length of the cellulose nanofibers may be measured, in the same manner as for the average fiber diameter, by visually measuring the length of each fiber. The median length of the measured values is taken as the average fiber length.

The water retention of the cellulose nanofibers is preferably 500 % or lower, more preferably 300 to 480 %. With a water retention of the cellulose nanofibers below 300 %, emulsion stability may not be obtained or a feeling of foreign matters may arise.

On the other hand, with a water retention of the cellulose nanofibers above 500 %, the water retention of the cellulose nanofibers themselves is high, which leads to emulsion stability, but homogenization of water drops or oil drops may be difficult.

The water retention of the cellulose nanofibers may be adjusted by, for example, selection, pretreatment, or defibration of the raw material pulp.

The water retention of the cellulose nanofibers is a value determined in compliance with JAPAN TAPPI No. 26 (2000).

The degree of crystallinity of the cellulose nanofibers is preferably 50 % or higher, more preferably 55 % or higher. On the other hand, the degree of crystallinity of the CNF is preferably 90 % or lower, more preferably 86 % or lower. With a degree of crystallinity of the CNF within the above range, the cellulose nanofibers are hardly affected physically or chemically by the materials other than cellulose used in the emulsification, and maintenance of the emulsion stability is facilitated.

The degree of crystallinity of the cellulose nanofibers may arbitrarily be adjusted by, for example, selection, pretreatment, or defibration of the raw material pulp.

The degree of crystallinity of the cellulose nanofibers is a value determined in compliance with JIS K 0131 (1996).

The pseudo particle size distribution curve of the cellulose nanofibers has preferably one peak. With one peak, the cellulose nanofibers have high uniformity in fiber length and fiber diameter, and a slurry of the cellulose fibers has excellent dewaterability.

The peak value of the cellulose nanofibers is, for example, 1 to 100 µm, preferably 3 to 80 µm, more preferably 5 to 60 µm.

The peak value of the cellulose nanofibers may be adjusted by, for example, selection, pretreatment, or defibration of the raw material pulp.

The peak value of the cellulose nanofibers is a value determined in compliance with ISO-13320 (2009). More specifically, first, a volume-based particle size distribution of an aqueous dispersion of the cellulose nanofibers is determined using a particle size distribution measuring apparatus (a laser diffraction/scattering-type particle size distribution measuring apparatus manufactured by SEISHIN ENTERPRISE CO., LTD.). Then the median diameter of the cellulose nanofibers is determined from this distribution, and this median diameter is taken as the peak value.

The pulp viscosity of the cellulose nanofibers is preferably 1 to 10 cps, more preferably 2 to 9 cps, particularly preferably 3 to 8 cps. The pulp viscosity is a viscosity of a solution of cellulose dissolved in a copper-ethylenediamine solution, and a higher pulp viscosity indicates higher degree of polymerization of cellulose. The pulp viscosity within the above-mentioned range leads to emulsion stability and restriction of a feeling of foreign matters.

The pulp viscosity is a value determined in accordance with TAPPI T 230.

The cellulose nanofibers obtained by the defibration may be dispersed in an aqueous medium and kept in the form of a dispersion, as needed, prior to mixing with other components. It is particularly preferred that the aqueous medium is entirely water (aqueous solution). However, part of the aqueous medium may be another liquid compatible with water. Such another liquid may be, for example, a lower alcohol having 3 or less carbon atoms.

The degree of polymerization of the cellulose nanofibers is preferably 300 or higher, more preferably 350 to 1800, particularly preferably 400 to 1700. A degree of polymerization below 300 may lead to deterioration of the emulsion stability and a feeling of foreign matters in the emulsion composition. Note that the degree of polymerization corresponds to the number of linked "two molecules of β-glucose", which is the minimum structural unit of cellulose. According to the present embodiment, the degree of polymerization is determined by a viscosity method using a copper-ethylenediamine solution.

The B-type viscosity of the dispersion of the cellulose nanofibers (1.5 % concentration) is preferably 1000 cps to 20000cps, more preferably 1000 to 10000 cps, particularly preferably 1000 to 5000 cps. The B-type viscosity of the dispersion within the above range facilitates mixing and dispersion with other components of the emulsion composition.

The B-type viscosity of the dispersion of the cellulose nanofibers (1.5 % solid content) is a value determined in compliance with JIS-Z8803: 2011 "Method for viscosity measurement of liquid". A B-type viscosity is a resistant torque in stirring a dispersion, and a higher value indicates more energy required for stirring.

The solid content of the cellulose nanofibers is preferably 0.1 % to 5.0 %, more preferably 0.3 to 4.0 %, particularly preferably 0.5 to 3.0 %. With a solid content of the cellulose nanofibers below 0.1 %, the fluidity is too high, which may impair dispersion stability after the emulsification. On the other hand, with a solid content of the cellulose nanofibers above 5.0 mass%, the fluidity is also remarkably low, which may cause difficulties in mixing with other components, deterioration of fluidity of the slurry per se, and incapability of homogeneous mixing.

The content of the cellulose nanofibers (2.0 mass% dispersion) in the emulsion composition is preferably 5 to 90 mass%, more preferably 5 to 85 mass%, particularly preferably 5 to 80 mass%. With a content of the cellulose nanofibers below 5 mass%, the emulsion stability is lost, which may lead to sedimentation or separation of the emulsion composition.

### (Other Components)

As a component enhancing the emulsion stability, in a separate addition to the cellulose nanofibers, a high-polymer component, such as xanthan gum, carboxyethyl cellulose, or carboxyvinyl polymer may be used. These high-polymer components increase viscosity of water to stabilize the emulsion composition.

When the emulsion composition according to the present embodiment is to be used as a component of a cosmetic composition, any of the following materials may preferably be contained as a functional component: various agents, for example, UV absorbers, such as paraaminobenzoic acid and derivatives thereof, homomethyl-7N-acetylalantoylanylate, butylmethoxybenzoylmethane, paramethoxycinnamate derivatives, including glyceryl di-paramethoxycinnamate-mono-2-ethylhexanoate or octylcinnamate, salicylate derivatives, including amylsalicylate, benzophenone derivatives, including 2,4-dihydroxybenzophenone, ethylhexyl dimethoxybenzylidene dioxoimidazoline propionate, liquid lanolin acetate, baikal skullcap root extract, and trianilino-p-carboethylhexyloxy-triazine; whitening components, such as arbutin, kojic acid, ascorbic acid and derivatives thereof including magnesium ascorbyl phosphate, glutathione, licorice root extract, clove extract, tea extract, astaxanthin, bovine placenta extract, tocopherol and derivatives thereof, tranexamic acid and salts thereof, azulene, and γ-hydroxybutyric acid; moisturizers, such as polyhydric alcohols including maltitol, sorbitol, glycerin, propylene glycol, 1,3-butylene glycol, polyethylene glycol, and glycol; organic acids and salts thereof including sodium pyrrolidone carboxylate, sodium lactate, and sodium citrate, hyaluronic acid and salts thereof including sodium hyaluronate, fermentation metabolites including yeast and hydrolysate of yeast extract liquid, yeast culture liquid, and lactic acid bacteria culture liquid, water-soluble proteins including collagen, elastin, keratin, and sericin, peptides and salts thereof including collagen hydrolysate, casein hydrolysate, silk hydrolysate, and sodium polyaspartate, sugars, polysaccharides, and derivatives thereof including trehalose, xylobiose, maltose, sucrose, glucose, and plant viscous polysaccharides, water-soluble chitin, chitosan, pectin, glycosaminoglycan and salts thereof including chondroitin sulfate and salts thereof, amino acids including glycine, serine, threonine, alanine, aspartic acid, tyrosine, valine, leucine, arginine, glutamine, and proline acid, glycoamino acid compounds including amino-carbonyl reaction products, plant extracts including aloe and horse chestnut extracts, trimethylglycine, urea, uric acid, ammonia, lecithin, lanolin, squalane, squalene, glucosamine, creatinine, and nucleic acid-related substances including DNA and RNA; thickeners, such as carboxymethyl cellulose, hydroxyethyl cellulose hydroxypropyl trimethylammonium chloride ether, ethyl cellulose, hydroxypropyl cellulose, methylhydroxypropyl cellulose, soluble starch, carboxymethyl starch, methyl starch, propylene glycol alginate, polyvinyl alcohol, polyvinylpyrrolidone, polyvinylmethyl ether, carboxyvinyl polymers, polyacrylic acids, methyl cellulose, hydroxyethyl cellulose, Arabic gum, xanthan gum, guar gum, locust bean gum, pyrus Cydonia seed, carrageenan, galactan, pectin, mannan, starch, dextran, succinoglucan, curdlan, hyaluronic acid, gelatin, casein, albumin, collagen, methoxyethylene-maleic anhydride copolymers, ampholytic methacrylate copolymers, poly N,N-dimethyl-3,4-dimethylene-pyrrolidinium chloride, polyacrylate copolymers, polyvinyl acetate, nitrocellulose, silicone resin, polyoxyethylene fatty acid esters including polyethylene glycol fatty acid esters and polyethyleneglycol distearate, polyoxyethylene methyl glycoside fatty acid esters including polyoxyethylene methyl glucoside dioleate, and α-olefin sulfonate including tetradecene sulfonate; chelating agents such as ethylene diamine tetraacetic acid and salts thereof, hydroxyethylenediamine triacetic acid and salts thereof, phosphoric acid, ascorbic acid, succinic acid, gluconic acid, polyphosphates, and metaphosphates; organic solvents, such as ethanol, propylene glycol, and 1,3-butylene glycol; antioxidants, such as butylhydroxy toluene, tocopherol, and phytic acid; antibacterial preservatives, such as benzoic acid and salts thereof, salicylic acid and salts thereof, sorbic acid and salts thereof, paraoxybenzoic acid alkyl esters (ethyl parabene, butyl parabene, and the like) and salts thereof, dehydroacetic acid and salts thereof, parachlorometacresol, hexachlorophene, boric acid, resorcin, tribromsalan, orthophenylphenol, chlorhexidine gluconate, thiram, photosensitizing dye No. 201, phenoxyethanol, benzalkonium chloride, benzethonium chloride, halocarbon, chlorhexidine chloride, trichlorocarbanilide, tocopherol acetate, zinc pyrithione, hinokitiol, phenol, isopropylmethylphenol, and 2,4,4-trichloro-2-hydroxyphenol, and hexachlorophene; blood circulation promoters, such as organic acids including citric acid, malic acid, tartaric acid, lactic acid, adipic acid, glutamic acid, aspartic acid, and maleic acid, Vitamins including Vitamin A and derivatives thereof, Vitamin B's including Vitamin B6 hydrochloride, Vitamin 6 tripalmitate, Vitamin B6 dioctanoate, and Vitamin B2 and derivatives thereof, Vitamin C's including ascorbic acid, ascorbyl sulfate, and ascorbyl phosphate, Vitamin E's including α-tocopherol, β-tocopherol, and γ-tocopherol, Vitamin D's, Vitamin H, and pantothenic acid, nicotinamide, benzyl nicotinate, γ-oryzanol, allantoin, glycyrrhizinic acid (salts), glycyrrhetinic acid and derivatives thereof, hinokitiol, mucidin, bisabolol, eucalyptol, thymolinositol, saponins (Quilaja saponin, azuki saponin, dishcloth gourd saponin, and the like), tranexamic acid, pantothelethyl ether, ethynylestradiol, cepharanthine, placenta extract, Japanese green gentian (Swertia japonica) extract, cepharanthine, Vitamin E and derivatives thereof, and γ-oryzanol; topical stimulant, such as capsicum tincture, ginger tincture, cantharides tincture, and benzyl nicotinate; nutrients, such as various vitamins and amino acids, e.g., Vitamin A's, Vitamin B family, Vitamin D family, Vitamin E, pantothenic acid, and Vitamin H; anti-inflammatory agents, such as glycyrrhetinic acid, glycyrrhizinic acid derivatives, carpronium chloride, nonylic acid vanillylamide, allantoin, azulene, aminocaproic acid, and hydrocortisone; astringent agents, such as zinc oxide, zinc sulfate, aluminum hydroxy allantoinate, aluminum chloride, zinc phenolsulfonate, and tannic acid; algefacients, such as menthol and camphor; antihistamines; silicone-based materials, such as polymeric silicone and cyclic silicone; antioxidants, such as tocopherols, BHA, BHT, gallic acid, and NDGA; natural extracts extracted or hydrolyzed with water, aqueous alcohol, or the like, from animals, plants, or microorganisms, or parts thereof, such as yeasts including Saccaromyces, filamentous bacteria, bacteria, bovine placenta, human placenta, human umbilical cord, yeasts, bovine collagen, milk-derived protein, wheat, soybean, bovine blood, porcine blood, cock's comb, chamomile, cucumber, rice, shea butter, white birch, tea, tomato, garlic, hamamelis, rose, dishcloth gourd, hop, peach, apricot, lemon, kiwifruit, Houttuynia cordata, pepper, sophora angustifolia, sorrel, candock, sage, siberian milfoil (Achillea sibirica), mallow, Cnidium officinale, Japanese green gentian (Swertia japonica), thyme, Angelica acutiloba, Japanese spruce, birch, field horsetail, horse chestnut, mother of thousands (Saxifraga stolonifera), arnica, lily, mugwort (Artemisia princeps), peony (Paeonia lactiflora), aloe, aloe vera, scutellaria root, phellodendron bark, carthami flos, safflower, gardenia fruit, Lithospermum root, jujube, citrus unshiu peel, ginseng, coix seed, job's tears, gardenia (Gardenia jasminoides), and sawara cypress; pigments; powder components, such as calcium carbonate, talc, kaolin, mica, sulfur, lauroyl lysine, fumed silica, titanium dioxide, zinc dioxide, red iron oxide, yellow iron oxide, black iron oxide, Nylon 12 powder, polymethylmethacrylate powder, polyethylene powder, and polystyrene powder; macromolecular additives, such as cationized cellulose, carboxyvinyl polymer, polyvinylpyrrolidone, polyvinylpyrrolidone-vinyl acetate copolymers, xanthan gum, and hydroxyethyl cellulose; flavoring agents; chelating agents; alkali, such as triethanolamine, potassium hydroxide, and borax; and antioxidants. The content of these components may be decided suitably depending on the purpose of the cosmetic composition.

### (Production Method)

The method for preparing an emulsion composition according to the present embodiment includes at least preparing an oil phase, preparing an aqueous phase, and emulsifying by mixing the oil phase and the aqueous phase.

According to the present embodiment, cellulose nanofibers are usually added in the form of a dispersion in at least one of the steps of preparing the aqueous phase and emulsifying, preferably in the step of preparing the aqueous phase. On the other hand, the emulsifier is added in at least any of the steps of preparing the oil phase, preparing the aqueous phase, and emulsifying, preferably in the step of preparing the oil phase.

The emulsification (step) may be conceived to be performed in various manners, and for obtaining an emulsion composition having excellent emulsion stability and sensation in use, for example, mechanical emulsification, D-phase emulsification, phase-inversion emulsification, liquid crystal emulsification, and amino acid gel emulsification may be employed. The mechanical emulsification may be performed using, for example, high-pressure homogenizer, colloid mill, nanomizer, microfluidizer, propeller stirrer, homomixer, homodisper, or the like. When a propeller stirrer, homomixer, homodisper, or the like is used, the rotational speed is 500 rpm or higher, preferably 800 rpm or higher, more preferably 2000 rpm or higher, particularly preferably 5000 rpm or higher.

### Examples

Test Examples of the present invention will be discussed.

### (Test Examples 1 to 4)

Into a 300 ml beaker, 132 g of ethylhexyl palmitate, dimethyl silicone oil, caster oil, or heptane was measured out as an oil component, then 2 g of polyoxyethylene sorbitan oleate was added as a surfactant (emulsifier), and the resulting mass was stirred at 300 rpm using a propeller stirrer. Next, 66 g of an unmodified CNF dispersion (2.0 % concentration) was added as an emulsion stabilizer, and emulsified. This emulsification was performed by stirring at 1000 rpm for 5 minutes using a propeller stirrer. In percent by mass, the oil component (ethylhexyl palmitate, dimethyl silicone oil, caster oil, or heptane) was 66 % (oil phase), the aqueous medium including the unmodified CNF (emulsion stabilizer) was 33 % (aqueous phase), and the surfactant (emulsifier) was 1 %.

As the test results, the emulsion stability immediately after stirring (initial emulsion stability) and the emulsion stability after 24 hours (emulsion stability after 24 hours) were determined as emulsion stability. The emulsions were evaluated as "O" when homogenously emulsified, as "Δ" when any aggregation was observed, and as "X" when the aqueous phase and the oil phase were separated. The same is applied to the subsequent Test Examples. Fig. 1 shows a photograph wherein the leftmost specimen is an example wherein homogeneous emulsion was obtained (O) (Test Example 1), the second from left is an example wherein aggregation was observed (Δ) (Test Example 11), and the third from left is an example wherein the aqueous phase and the oil phase were separated (X) (Test Example 14). In Test Examples 1 to 4, no emulsion compositions with any oil components were separated.

### (Test Example 5)

Into a 300 ml beaker, 128 g of ethylhexyl palmitate was measured out as an oil component, to which a stirred mixture of 60 g of an unmodified CNF dispersion (2.0 % concentration) as an emulsion stabilizer and 12 g of polyoxyethylene sorbitan oleate as a surfactant was introduced for moisture adjustment, and emulsified. This emulsification was performed by stirring at 1000 rpm for 5 minutes using a propeller stirrer. The emulsion composition was not separated.

### (Test Example 6)

Into a 300 ml beaker, 128 g of ethylhexyl palmitate was measured out as an oil component, to which a stirred mixture of 60 g of an unmodified CNF dispersion (2.0 % concentration) as an emulsion stabilizer and 12 g of polyoxyethylene sorbitan monolaurate as a surfactant was introduced for moisture adjustment, and emulsified. This emulsification was performed by stirring at 1000 rpm for 5 minutes using a propeller stirrer. The emulsion composition was not separated.

### (Test Example 7)

Into a 300 ml beaker, 149 g of ethylhexyl palmitate was measured out as an oil component, to which 2 g of polyoxyethylene sorbitan oleate as a surfactant (emulsifier) was introduced, and stirred at 300 rpm using a propeller stirrer. Next, 49 g of an unmodified CNF dispersion (2.0 % concentration) as an emulsion stabilizer was added and emulsified. This emulsification was performed by stirring at 1000 rpm for 5 minutes using a propeller stirrer. The emulsion composition was not separated.

### (Test Example 8)

Into a 300 ml beaker, 99 g of ethylhexyl palmitate was measured out as an oil component, to which 2 g of polyoxyethylene sorbitan oleate as a surfactant (emulsifier) was introduced. On the other hand, 49 g of an unmodified CNF dispersion (2.0 % concentration) as an emulsion stabilizer and 50 g of purified water were mixed for moisture adjustment, and stirred at 300 rpm using a propeller stirrer. The mass resulting from this stirring was introduced into the oil component prepared above, and emulsified. This emulsification was performed by stirring at 1000 rpm for 5 minutes using a propeller stirrer. The emulsion composition was not separated.

### (Test Example 9)

Into a 300 ml beaker, 66 g of ethylhexyl palmitate and 66 g of dimethyl silicone were measured out and mixed as oil components, and stirred at 300 rpm using a propeller stirrer. Next, 66 g of an unmodified CNF dispersion (2.0 % concentration) as an emulsion stabilizer was added to the oil components, and finally 2 g of polyoxyethylene sorbitan oleate as a surfactant (emulsifier) was introduced into the oil components and emulsified. This emulsification was performed by stirring at 1000 rpm for 5 minutes using a propeller stirrer. The emulsion composition was not separated.

### (Test Example 10)

Into a 300 ml beaker, 120 g of ethylhexyl palmitate was measured out as an oil component, to which 30 g of polyoxyethylene sorbitan monolaurate as a surfactant (emulsifier) was introduced, and stirred at 300 rpm using a propeller stirrer. Next, 50 g of an unmodified CNF dispersion (2.0 % concentration) as an emulsion stabilizer was added to the oil component and emulsified. This emulsification was performed by stirring at 1000 rpm for 5 minutes using a propeller stirrer. The emulsion composition was not separated.

### (Test Example 11)

Into a 300 ml beaker, 132 g of ethylhexyl palmitate was measured out as an oil component, to which 2 g of polyoxyethylene sorbitan oleate as a surfactant (emulsifier) was introduced, and stirred at 300 rpm using a propeller stirrer. Next, 66 g of TEMPO-oxidized CNF (2.0 % concentration) as an emulsion stabilizer was added and emulsified. This emulsification was performed by stirring at 1000 rpm for 5 minutes using a propeller stirrer. Even immediately after the stirring, homogenous dispersion was not observed and aggregates were formed.

### (Test Example 12)

Into a 300 ml beaker, 132 g of ethylhexyl palmitate was measured out as an oil component, to which 2 g of polyoxyethylene sorbitan oleate as a surfactant (emulsifier) was introduced, and stirred at 300 rpm using a propeller stirrer. Next, 66 g of purified water was added (conditions not containing the emulsion stabilizer (CNF)) and emulsified. This emulsification was performed by stirring at 1000 rpm for 5 minutes using a propeller stirrer. The initial dispersibility was good, but after 24 hours, the aqueous phase and the oil phase were separated.

### (Test Example 13)

Into a 300 ml beaker, 132 g of caster oil was measured out as an oil component, and then 66 g of an unmodified CNF dispersion (2.0 % concentration) as an emulsion stabilizer was added and emulsified. This emulsification was performed by stirring at 1000 rpm for 5 minutes using a propeller stirrer. In this test, no emulsifier was used. The initial dispersibility was good, but after 24 hours, the aqueous phase and the oil phase were separated.

### (Test Example 14)

Into a 300 ml beaker, 120 g of ethylhexyl palmitate was measured out as an oil component, to which 40 g of polyoxyethylene sorbitan monolaurate as a surfactant (emulsifier) was introduced, and stirred at 300 rpm using a propeller stirrer. Next, 40 g of an unmodified CNF dispersion (2.0 % concentration) as an emulsion stabilizer was added and emulsified. This emulsification was performed by stirring at 1000 rpm for 5 minutes using a propeller stirrer. Even immediately after the stirring, homogenous dispersion was not observed, and the initial dispersion tended to separate, resulting in phase separation.

**Table 1**

| Category | Name | Concen-tration | Test Example 1 | Test Example 2 | Test Example 3 | Test Example 4 | Test Example 5 | Test Example 6 | Test Example 7 | Test Example 8 | Test Example 9 | Test Example 10 | Test Example 11 | Test Example 12 | Test Example 13 | Test Example 14 |
|---|---|---|---|---|---|---|---|---|---|---|---|---|---|---|---|---|
| Oil phase | Ethylhexyl palmitate | 100% | 66% | | | | 64% | 50% | 75% | 50% | 33% | 60% | 66% | 66% | | 60% |
| | Dimethyl silicone | 100% | | 66% | | | | | | | 33% | | | | | |
| | Caster oil | 100% | | | 66% | | | | | | | | | | 66% | |
| | Heptane | 100% | | | | 66% | | | | | | | | | | |
| Aqueous phase | Unmodified mechanically processed CNT aqueous soluton | 2% | 33% | 33% | 33% | 33% | 30% | 50% | 25% | 24% | 33% | 25% | | | 34% | 20% |
| | TEMPO-oxidized CNF aqueous solution | 2% | | | | | | | | | | | 33% | | | |
| | Purified water | | | | | | | | | 25% | | | | 33% | | |
| Surfactant | Polyoxyethylene sorbitan monolaurate | 100% | | | | | | 1% | | | | 15% | | | | 20% |
| | Polyoxyethylene sorbitan oleate | 100% | 1% | 1% | 1% | 1% | 6% | | 1% | 1% | 1% | | 1% | 1% | | |
| | | Total | 100% | 100% | 100% | 100% | 100% | 100% | 100% | 100% | 100% | 100% | 100% | 100% | 100% | 100% |
| Evaluation result | Initial emulsion stability | | ○ | ○ | ○ | ○ | ○ | ○ | ○ | ○ | ○ | ○ | Δ | ○ | ○ | × |
| | Emulsion stability after 24 hrs | | ○ | ○ | ○ | ○ | ○ | ○ | ○ | ○ | ○ | ○ | Δ | × | × | × |

The CNF used are as follows:
Unmodified mechanically processed CNF: mechanically processed CNF manufactured by DAIO PAPER CORPORATION
TEMPO-oxidized CNF: manufactured by DKS CO., LTD.

### (Consideration)

By comparing Test Examples 1 and 12, it is seen that when the oil component and the emulsifier were mixed, emulsion state was temporarily observed but not stable over time, but by adding CNF, the stability over time was improved.

By comparing Test Examples 3 and 13, it is seen that when the oil component and the CNF were mixed, emulsion state was temporarily observed, but the stability over time was poor without the emulsifier.

From the test results, it is understood that the unmodified CNF was suitable for adding as an emulsion stabilizer for emulsifying and stabilizing an oil component and an aqueous medium. In this regard, it is also understood that a modified CNF is not preferred as an emulsion stabilizer.

By comparing Test Examples 10 and 14, it is seen that the content of the emulsifier is preferably less than 20 %.

### Industrial Applicability

The present invention is industrially applicable as an emulsion composition, a cosmetic composition, and a method for preparing an emulsion composition.

## Claims

1. An emulsion composition comprising: an oil component; an aqueous dispersion medium; an emulsifier, and cellulose nanofibers,
wherein the cellulose nanofibers are an emulsion stabilizer, and formed of defibrated unmodified cellulose, and
an amount of the emulsifier is less than 20 mass% of a total amount of the emulsion composition.

2. The emulsion composition according to claim 1,
wherein the emulsifier is a nonionic surfactant comprising at least one of a fatty acid or a fatty acid ester.

3. The emulsion composition according to claim 1 to 2,
wherein a content of the cellulose nanofibers (in terms of absolute dry solid) is more than 2.0 mass% of the content of the emulsifier.

4. A cosmetic composition, comprising the emulsion composition according to any one of claims 1 to 3.

5. A method for preparing an emulsion composition, comprising:
preparing an oil phase, preparing an aqueous phase, and emulsifying by mixing the oil phase and the aqueous phase,
wherein cellulose nanofibers obtained by defibrating unmodified cellulose are added in at least one of the preparing of an aqueous phase and the emulsifying, and
an emulsifier is added in at least any of the preparing of an oil phase, the preparing of an aqueous phase, and the emulsifying.
